# EUROPEAN PATENT APPLICATION

(11) **EP 1 992 341 A1**
(43) Date of publication of application: **19.11.2008**
(21) Application number: 07290635.7
(22) Date of filing: 16.05.2007
(51) Int. Cl.: A61K 31/198, A61P 3/10

(54) **New pharmaceutical compositions comprising a thyroid hormon and their therapeutic use**

(71) Applicant: Université Joseph Fourier, 38041 Grenoble Cédex 9 (FR); INSERM (Institut National de la Santé et de la Recherche Médicale), 75654 Paris Cedex 13 (FR); Centre National de la Recherche Scientifique, 75794 Paris Cedex 16 (FR)
(72) Inventor: The designation of the inventor has not yet been filed
(74) Representative: Grosset-Fournier, Chantal Catherine

(57) **Abstract**

The present invention relates to a pharmaceutical composition comprising, as active substance, at least one hormone chosen among 3',5',3-triiodothyronine (rT3), a rT3 derived hormone, or a precursor of rT3, such as T4 in association with a molecule susceptible to promote the formation of rT3, in association with a pharmaceutically acceptable vehicle.

## Description

The present invention relates to new pharmaceutical compositions comprising a thyroid hormone and their therapeutic use.

Thyroid hormones have been known for a long time. The thyroid hormone family consists in T4 hormone and the derived iodothyronines resulting from successive monodeiodinations of T4. The pathways of the deiodination cascade of T4 have been described by Hulbert A.J. (Biol. Rev., 2000). T4 gives T3 via an outer ring 5'-deiodination or rT3 via an inner ring 5'-deiodination. T3 results in 3,5-T2 via an outer ring 5'-deiodination or 3,3'-T2 via an inner ring 5'-deiodination. rT3 results in 3,3'-T2 via an outer ring 5'-deiodination or 3',5'-T2 via an inner ring 5'-deiodination. 3-T1 is obtained via an inner ring 5'-deiodination from 3,5-T2 or via an outer ring 5'-deiodination from 3,3'-T2. 3'-T1 is obtained via an inner ring 5'-deiodination from 3,3'-T2 or via an outer ring 5'-deiodination from 3',5'-T2.

For information, table 1 indicates the formula of several members of the thyroid hormone family.

**Table 1: Formula of iodothyronine hormones**

| | | |
|---|---|---|
| 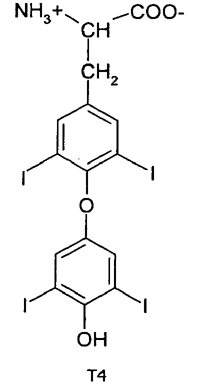 | 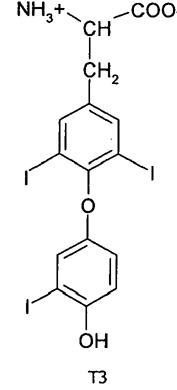 | 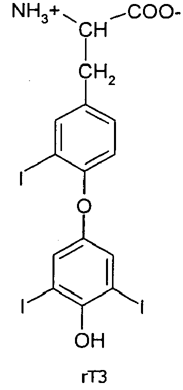 |
| 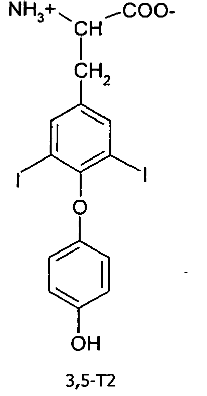 | 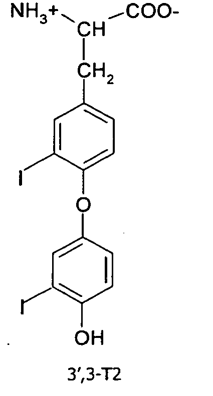 | 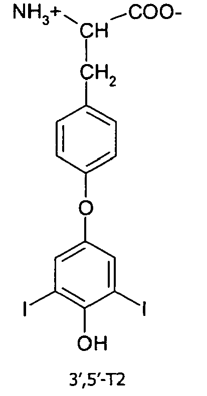 |
| 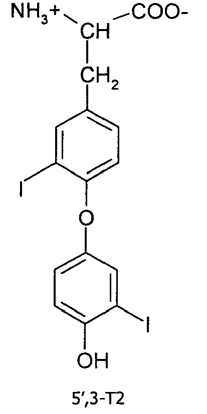 | 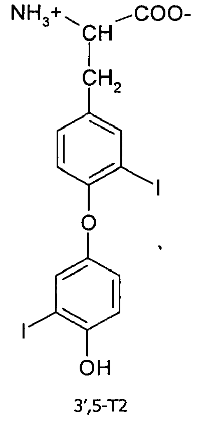 | 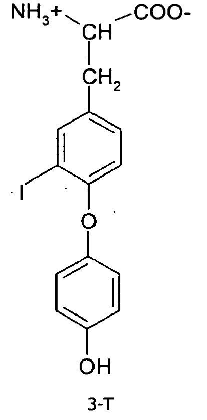 |
| 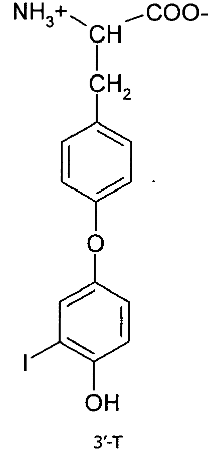 | 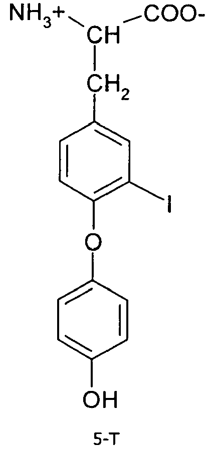 | 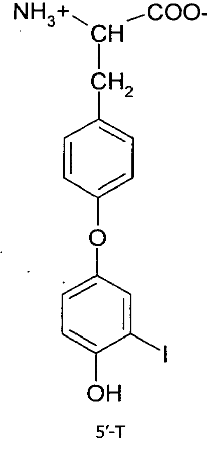 |

The known effects of thyroid hormones, particularly of the T3 hormone, result mainly from the binding to two nuclear receptors of the thyroid hormones, TRα-1 and TRβ-1 belonging to the family of nuclear receptors TR-α and TR-β, which are supposed to have different effects. These receptors are thought to be highly specific towards T3, particularly relating to the number of iodine and the spatial arrangement (Bolger et al., J. Biol. Chem., 1980; Koerner et al., J. Biol. Chem., 1975; Dietrich et al., J. Med Chem., 1977) . Since the discovery of the thyroid nuclear receptors, most of scientists have focused on the effects of transcriptional changes of thyroid hormones.

T3 hormone binds very efficiently to the nuclear receptors, whereas the T4 hormone binds less efficiently. The hormones derived from T4 and T3 do not bind to the nuclear receptors (Koerner et al., J. Biol. Chem., 1975; Lazar, Endocrine Rev., 1993; Hulbert, Bio. Rev., 2000; Oppenheimer, Biochimie, 1999; Yen, Physiol. Rev., 2001).

The use of T3 hormone for treating obesity is well known by the man skilled in the art. However, its use has been highly limited because of serious side effects of T3 hormone, particularly cardiac side effects. The treatment of hypothyroidism lies on T3, which can be used directly or produced *in vivo* by the transformation of its very little active precursor, the T4 hormone (Yen, Physiol. Rev., 2001). T3 is known as the real active thyroid hormone.

The effects induced by thyroid hormones, such as T3, via the nuclear receptor pathway are physiologically important effects observed at very low concentrations. These effects are often deleterious when T3 is administered to subjects that do not suffer from hypothyroidism. These effects can be considered as "hyperthyroidic effects" linked to the nuclear receptor pathway.

The international application WO2005/009433 and the corresponding scientific paper (Lanni et al., The FASEB Journal, 2005) have disclosed an effect of the 3,5-T2 on energetic metabolism. More particularly, normal rats receiving a high-fat diet and treated with a daily peritoneal injection of 3,5-T2 gained less weight and had less fat deposit than untreated rats. The 3,5-T2 hormone was thus proposed for the treatment of obesity and related pathologies.

The rT3 hormone is generally considered as an inactive hormone and was thought to represent the inactivation pathway of thyroid hormones (Yen, Physiol. Rev., 2001). Thus, increased rT3 plasmatic concentrations are often found in low T3 syndrome. Recently, cerebral effects of rT3 have been disclosed in the establishment and structuring of astrocytes (Farwell et al. Endocrinology, 2006).

In prior art, it has never been disclosed that thyroid hormones may have effects on insulin and glycemia.

Diabetes is a chronic disease characterized by a hyperglycemia.

Type 1 diabetes results from the destruction of the pancreatic β cells secreting insulin. Treatment of type 1 diabetes particularly consists in the administering of insulin.

Type 2 diabetes is more frequent than type 1 diabetes in the population and is generally associated to obesity. Type 2 diabetes is characterized by two interdependent abnormalities: an insulin-resistance and a reduced production of insulin by response to glycemia.

Treatments of type 2 diabetes particularly consist in using an agonist drug of insulin or an agonist of insulin secretion by the beta cells, in reducing the glycemia and the weight of the diabetic patients.

Obesity is one of the major public health concerns in developed countries as well as in developing countries. The mechanisms involved in obesity are not really understood. Factors involved in obesity are particularly alimentation (fat and sweet diets) and environment conditions (physical activity, social environment, food availability).

More efficient and more appropriate treatments (particularly in term of side effects, comfort of patients such as frequency of use and administering route) are needed against chronic diseases such as diabetes, obesity and dyslipidemia.

One aim of the present invention is to provide new pharmaceutical compositions comprising a thyroid hormone as active substance.

Another aim of the invention is to provide new pharmaceutical compositions for the treatment of metabolic disorders that do not induce hyperthyroidism.

Another aim of the invention is to provide a new therapeutic class of drugs for the treatment of diabetes.

Another aim of the invention is to provide a combination product for a simultaneous, separate or sequential use intended for the treatment of diabetes.

The present invention relates to a pharmaceutical composition comprising, as active substance, at least one hormone chosen among:
- 3',5',3-triiodothyronine (rT3),
- a rT3 derived hormone, such as 3',3-diiodothyronine (3',3-T2), 3',5'-diiodothyronine (3',5'-T2), 5',3-diiodothyronine (5',3-T2), 3'-iodothyronine (3'-T), 5'-iodothyronine (5'-T) or 3-iodothyronine (3-T), or
- a precursor of rT3, such as T4 in association with a molecule susceptible to promote the formation of rT3,
in association with a pharmaceutically acceptable vehicle.

Contrary to the teaching of the prior art presenting rT3 as inactive, the Inventors have found that the rT3 hormone can be used as a drug, as well as its derived hormones.

Furthermore, contrary to the effects of other thyroid hormones such as T3, the effects of the hormones according to the invention seem not to involve the nuclear receptor pathway since rT3 is know to have a very little affinity to TH receptors.

The rT3 hormone and the rT3 derived hormones are the physiological forms of thyroid hormones that are inactive for the treatment of hypothyroidism or as hyperthyroidism inducer. Contrary to 3,5-T2, these hormones can not be obtained via T3, which is the active thyroid hormone.

According to the Inventors, rT3, a rT3 derived hormone or a rT3 precursor are shown for the first time to have an energetic activity and to have an effect on glycemia and on insulin sensitivity as well as plasma concentrations.

The Inventors propose that the use of rT3 and its derived hormone is the physiological way to obtain beneficial metabolic effects without inducing hyperthyroidism.

Futhermore, the Inventors propose that rT3 has beneficial effect only on the glycemia of diabetic subjects and has no hypoglycemic effect on non diabetic subjects (see Examples section).

According to the present invention, the term "3',5',3-triiodothyronine" refers to reverse T3 or rT3.

By the expression "rT3 derived hormone", one means any compound that has at least one iodine susceptible to be obtained from rT3, particularly by removing one or several iodines, via natural occurring and / or artificial ways.

By "natural occurring way", it is particularly meant that the rT3 derived hormone is obtained via enzymes such as the iodothyronine deiodinases that remove one or several iodines from rT3. Several biological reactions may be needed to obtain the desired derived hormone.

By the expression "via an artificial way", it is particularly meant that the rT3 derived hormone is obtained via chemical synthesis, biochemical synthesis or recombinant technology.

The preferred rT3 derived hormones are diiodothyronines and iodothyronines. The preferred rT3 derived hormones are 3',3-diiodothyronine, 3',5'-diiodothyronine, 5',3-diiodothyronine, 3'-iodothyronine, 5'-iodothyronine or 3-iodothyronine.

By the expression "a precursor of rT3", one means any compound susceptible to give rT3. The precursor of rT3 may be a natural hormone, a synthesis or recombinant hormone, or a modified hormone.

By the expression "natural hormone", one means a hormone found in a living being, such as an animal or a human being, and which is purified and isolated from said living being.

By the expression "synthesis or recombinant hormone", one means a hormone obtained by chemical or biochemical synthesis or recombinant technology.

By the expression "modified hormone", one means a hormone which is chemically modified to add functional groups. Said functional groups may modify the activity of said hormone or protect said hormone from degradation.

In an advantageous embodiment of the invention, the precursor of rT3 is the T4 hormone, also called "thyroxine".

The rT3 precursor is preferentially used in association with a molecule susceptible to promote the formation of rT3. The use of the precursor of rT3 and said molecule can be simultaneous, separate or sequential.

Particularly, when the T4 hormone is used as rT3 precursor, said molecule susceptible to promote the formation of rT3 is:
- a thyronine deiodinase that allows the preferential formation of the rT3 hormone instead of the T3 hormone, or an agonist of said thyronine deiodinase, or
- an antagonist of a deiodinase that allows the preferential formation of the T3 hormone instead of the rT3 hormone.

The present invention particularly relates to a pharmaceutical composition as defined above, wherein said active substance is rT3.

The present invention particularly relates to a pharmaceutical composition as defined above, in a suitable form for the release of about 0.01 µg/kg/day to about 250 µg/kg/day, particularly about 0.01 µg/kg/day to about 25 µg/kg/day, particularly about 0.1 µg/kg/day to about 15 µg/kg/day of active substance, more particularly about 0.1 µg/kg/day to about 5 µg/kg/day of active substance, most particularly about 0.1 µg/kg/day to 1 µg/kg/day of active substance.

The dosage of active substance particularly depends on the administration route, which is easily determined by the man skilled in the art.

The present invention further relates to pharmaceutical composition as defined above, comprising by dosage unit about 5 µg to about 1.5 g of active substance, particularly about 75 mg to about 750 mg of active substance, to be released in a lapse of time corresponding to the above-mentioned values of the ranges in µg/kg/day or mg/kg/day for a 70kg human.

As an example, for the treatment of a 70 kg human, the dosage will be:
- about 5 µg to about 150 mg, particularly about 5 µg to about 15 mg, particularly about 50 µg to about 10 mg, particularly about 50 µg to about 3 mg, most particularly about 50 µg to about 500 µg of active substance to achieve an eight day treatment,
- about 20 µg to about 500 mg, particularly about 20 µg to about 50 mg, particularly about 200 µg to about 30 mg, particularly about 200 µg to about 10 mg, most particularly about 200 µg to about 2 mg of active substance to achieve a thirty day treatment,
- about 60 µg to about 1.5 g, particularly about 60 µg to about 150 mg, particularly about 600 µg to about 100 mg, particularly about 600 µg to about 30 mg, most particularly about 600 µg to about 6 mg of active substance to achieve a ninety day treatment.

By the expression "dosage unit", one means the quantity of active substance comprised in one drug unit.

Depending on the administration route and on the formulation of the pharmaceutical composition, the active substance comprised in the dosage unit can be released quickly or continuously over a period of time. The pharmaceutical composition can also be a slow-release drug.

Pharmaceutical compositions of the invention may be administered in a partial dose or a dose one or more times during a 24 hour period. Fractional, double or other multiple doses may be taken simultaneously or at different times during a 24 hour period.

In an advantageous embodiment, the pharmaceutical composition of the invention is administered in a unique dose, which allows a continuous release for a period of time of at least 24h, preferably at least one week, more preferably at least one month, most preferably at least two months, in particular three months.

The present invention also relates to the use of at least one hormone chosen among:
- 3',5',3-triiodothyronine (rT3),
- a rT3 derived hormone, such as 3',3-diiodothyronine, 3',5'-diiodothyronine, 5',3- diiodothyronine, 3'-iodothyronine, 5'-iodothyronine, or 3-iodothyronine, or
- a precursor of rT3, such as T4, in association with a molecule susceptible to promote the formation of rT3,
for the preparation of a drug intended for the treatment of:
- hyperglycemia, insulin resistance, beta pancreatic cell insufficiency, or related pathologies,
- pathologies wherein the cholesterol and / or triglycerides plasma concentrations are higher than the normal concentrations, or dyslipidemia, or
- pathologies related to overweight or related to an excess of fat deposit.

In an advantageous embodiment, the present invention relates to the use as defined above, for the preparation of a drug intended for the treatment of type 1 and type 2 diabetes, hyperglycemia, insulin resistance, beta pancreatic cell insufficiency, or related pathologies.

The Inventors have shown for the first time that rT3, a rT3 derived hormone or a rT3 precursor are capable of reducing glycemia and insulin plasmatic concentrations.

Hyperglycemia is characterized by fasting glucose concentrations higher that 1.1 g/l (or 110 mg/dl or 5.5 mmol/l), particularly higher than 1.20 g/L. The use of rT3, a rT3 derived hormone or a rT3 precursor allows reducing glycemia to normal concentrations.

By the expression "normal concentrations of glucose", one means glucose plasmatic concentration comprised from 4.4 mmol/l to 5.5 mmol/l, "abnormal" blood glucose is defined by fasting plasma glucose >5.55 mmol/l and diabetes by fasting plasma glucose >6.1 mmol/l (Meggs et al., Diabetes, 2003).

Glycemia is assessed by classical blood tests using the glucose oxidase method as reference (Yeni-Komshian et al., Diabetes Care, 2000, p171-175; Chew et al., MJA, 2006, p445-449; Wallace et al., Diabetes Care, 2004, p1487-1495).

The use of rT3, a rT3 derived hormone or a rT3 precursor also improves insulin resistance.

Insulin resistance is characterized by insulin plasmatic concentrations higher than 8 mU/l or 60 pmol/l (Wallace et al., Diabetes Care, 2004, p1487-1495).

Insulin resistance is the condition in which normal amounts of insulin are inadequate to produce a normal response from fat, muscle and liver cells, *i.e.* a resistance to the physiological action of insulin.

It is defined as the lowest quartile of measures of insulin sensitivity (e.g. insulin stimulated glucose uptake during euglycaemic clamp) or highest quartile of fasting insulin or homeostasis model assessment (HOMA) insulin resistance index in the population studied (Alberti et al. "Definition, diagnosis and classification of diabetes mellitus and its complications. Part 1: Diagnosis and classification of diabetes mellitus, provisional report of a WHO consultation", Diabetic Med, 1998, p539-553; Wallace et al., Diabetes Care, 2004, p1487-1495).

The use of the above-mentioned active substances allows reducing insulin plasmatic concentrations to normal concentrations, increasing the sensitivity to insulin and improving the metabolism of glucose and lipids.

By the expression "normal concentrations of insulin", one means insulin plasmatic concentration comprised from 5 to 8 mU/l (36 to 60 pmol/l).

Insulin concentration is assessed by classical blood tests (RIA assay with human antibody; Yeni-Komshian et al., Diabetes Care, 2000, p171-175; Chew et al., MJA, 2006, p445-449; Wallace et al., Diabetes Care, 2004, p1487-1495).

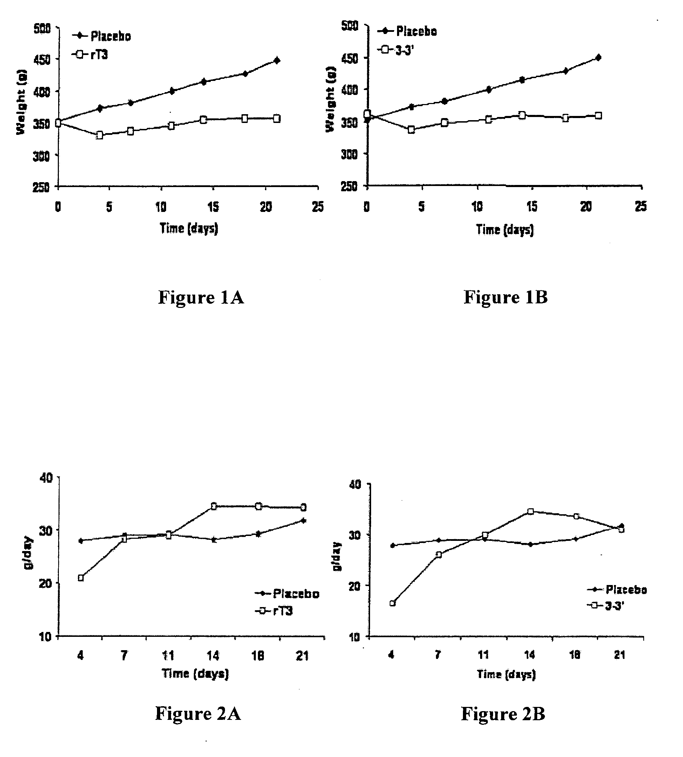

Sensitivity to insulin can be assessed by the HOMA (Homeostasis Model Assessment) method (Wallace et al., Diabetes Care, 2004, p1487-1495, see Figure 2 on page 1489).

Surprisingly, the use of the above-mentioned active substances seems to improve pancreatic β cells survival, and thus the regeneration of said insulin secreting cells.

The regeneration of said cells is evaluated through the measurement of insulin concentration (RIA assay with human antibody; Yeni-Komshian et al., Diabetes Care, 2000, p171-175; Chew et al., MJA, 2006, p445-449; Wallace et al., Diabetes Care, 2004, p1487-1495).

Results obtained on ZDF rats show that treatment with rT3 induced decreasing glucose concentration and increasing plasmatic insulin concentration.

In Goto-Kakizaki (GK) rats, a genetic model of type 2 diabetes, there is a restriction of the β cell mass as early as fetal age, which is maintained in the adult animal. The restriction of the β cell mass can be considered as a crucial event in the sequence leading to overt diabetes in this model. In the GK model, the regeneration of β cells occurs with a lower efficiency as compared to non-diabetic Wistar rats. This defect in the GK rats is both the result of genetic predisposition contributing to an altered β cells neogenesis potential and environment factors, such as chronic hyperglycemia, leading to a reduced β cell proliferative capacity specific to the adult animals. These results are described in Movassat et al., Diabetologia, 1997, p916-925 and in Plachot et al., Histochem Cell Biol., 2001, p131-139, the entire contents of which are incorporated herein by reference.

Assuming that a chronic hyperglycemia induced a destruction of pancreatic β cells and thus a decreased secretion of insulin, restored normal insulin concentrations could mean that the β cells are regenerated.

The β cells functional mass can be correlated to the level of insulin secretion through the HOMA method. On animal models, the man skilled in the art can envision the direct evaluation of pancreas mass.

According to another embodiment, the present invention relates to the use as defined above, for the preparation of a drug intended for the treatment of.pathologies wherein the cholesterol and / or triglyceride plasmatic concentrations are higher than the normal concentrations, or dyslipidemia, or pathologies related to overweight or related to an excess of fat deposit.

A cholesterol concentration higher than the normal concentrations means a plasmatic concentration higher than 2.5 g/l.

A triglyceride concentration higher than the normal concentrations means a plasmatic concentration higher than 2 g/l.

Dyslipidemia is characterized by a triglyceride concentration higher than 1.7 mmol/l and/or a HDL-cholesterol level lower than 1 mmol/l (men) or 1.3 mmol/l (women) (Chew, MJA, 2006, p445-449, see table entitled "Clinical definitions of the metabolic syndrome").

An excess of fat deposit is characterized by a body mass index: (weight, kg / height², m²) higher than 25 kg/m² and obesity is characterized by a body mass index higher than 30 kg/m².

The invention further relates to the use as define above, for the preparation of a drug intended for the treatment of pathologies chosen among diabetes, particularly type 1 or 2 diabetes, beta pancreatic cell insufficiency, obesity, overweight or related pathologies, hypercholesterolemia, hypertriglyceridemia, dyslipidemia, alcoholic and non alcoholic hepatic steatosis, atherosclerosis, hepatopathies associated to a dysmetabolism, cholecystopathies, deposit of subcutaneous fat, particularly cellulite or vasomotor rhinitis.

In an advantageous embodiment, the invention related to the uses as defined above, wherein said hormone is rT3.

The present invention particularly relates to a pharmaceutical composition as defined above, wherein said pharmaceutically acceptable vehicle refers to pharmaceutically acceptable solid or liquid, diluting or encapsulating, filling or carrying agents, which are usually employed in pharmaceutical industry for making pharmaceutical compositions.

The present invention relates to a pharmaceutical composition as defined above, suitable for an administration via an oral, intravenous, intramuscular, subcutaneous, transcutaneous, nasal, intraperitoneal, sublingual, or rectal route.

In the oral route, drugs are administered orally, particularly under the shape of tablets, coated tablets, pills, syrup or elixirs, dragees, troches, lozenges, aqueous or oily suspensions, liquid solutions, dispersible powders or granules, emulsions, hard or soft capsules.

In the intravenous route or systemic route, the drug can be administered in the bloodstream by a single injection or via a continuous infusion, eventually via a pump. The intravenous injection is not the easiest way for obese patients.

In the cutaneous route, drugs are applied to the skin. The formulation may be an ointment, a cream, a lotion, a solution, a powder or a gel.

In the subcutaneous route, the drug can be injected directly into fatty tissue just beneath the skin or the drug can be included in capsules that are inserted under the skin.

In the transcutaneous route, the drug passes through the skin to the bloodstream without injection. Particularly, the drug is comprised in a patch applied on the skin. Concerning patches formulation, the drug can be mixed with a chemical, such as alcohol, to enhance skin penetration.

The dosage forms include immediate release, extended release, pulse release, variable release, controlled release, timed release, sustained release, delayed release, long acting, and combinations thereof.

The dosage forms include, without limitation, tablets, multi-layer tablets, bi-layer tablets, chewable tablets, quick dissolve tablets, effervescent tablets, syrup, suspensions, emulsions, capsules, soft gelatin capsules, hard gelatin capsules, lozenges, chewable lozenges, beads, powders, granules, particles, microparticles, dispersible granules, cachets, creams, topicals, patches, implants, injectables (including subcutaneous, intramuscular, intravenous, and intradermal), infusions.

In an advantageous embodiment of the invention, the pharmaceutical composition is suitable for a transcutaneous, particularly by the means of patches.

In an advantageous embodiment, the administration of the pharmaceutical composition avoids partially that the drug passes through liver, which is susceptible of an important degradation of the hormones.

In another advantageous embodiment of the invention, the pharmaceutical composition is suitable for a subcutaneous administration, particularly by the means of a capsule injected beneath the skin.

The present invention also relates to a pharmaceutical composition as defined above, wherein said pharmaceutically acceptable vehicle allows a continuous, preferably constant, release, of said active substance, the active substance being chosen among:
- 3',5',3-triiodothyronine (rT3),
- a rT3 derived hormone, such as 3',3-diiodothyronine (3',3-T2), 3',5'-diiodothyronine (3',5'-T2), 5',3-diiodothyronine (5',3-T2), 3'-iodothyronine (3'-T), 5'-iodothyronine (5'-T) or 3-iodothyronine (3-T), or
- a precursor of rT3, such as T4 in association with a molecule susceptible to promote the formation of rT3.

The continuous, preferably constant, release of the active substance allows obtaining:
- increased effects on metabolic disorders as compared to results obtained *via* another administration mode, or
- newly observed effects on metabolic disorders on animal models on which there were previously no positive results.

By the expression "continuous release", one means a continuous release of the drug over at least 24 hours, preferably at least one month, most preferably at least two months, in particular three months.

By the expression "constant release", one means a continuous release of the drug over at least 24 hours, preferably at least one month, most preferably at least two months, in particular three months, the quantity of released drug/ time unit being essentially constant.

A continuous and constant release is for example achieved by using patches or capsules injected under the skin.

In another embodiment, rT3, the rT3 derived hormone and the rT3 precursor of the invention are used in a simultaneous, separate or sequential combination with another thyroid hormone, such as 3,5-T2 or 3',5-T2.

The present invention also relates to a product comprising:
- at least one hormone chosen among 3',5',3-triiodothyronine (rT3), a rT3 derived hormone, such as 3',3-diiodothyronine, 3',5'-diiodothyronine, 5',3-diiodothyronine, 3'-iodothyronine, 5'-iodothyronine, or 3-iodothyronine, or a rT3 precursor, such as T4 in association with a molecule susceptible to promote the formation of rT3,
- at least one active substance activating the pancreatic secretion of insulin, particularly chosen among antidiabetic oral drugs, or susceptible of slowing the digestive absorption of glucose,
as a combination product for a simultaneous, separate or sequential use intended for the treatment of diabetes.

The present invention also relates to nutraceutics or food compositions comprising at least one hormone chosen among:
- 3',5',3-triiodothyronine (rT3),
- a rT3 derived hormone, such as 3',3-diiodothyronine, 3',5'-diiodothyronine, 5',3- diiodothyronine, 3'-iodothyronine, 5'-iodothyronine or 3-iodothyronine, or
- a precursor of rT3, such as T4 in association with a molecule susceptible to promote the formation of rT3.

The present invention also relates to a method for improving meat quality, in particular pork meat quality, by controlling the ratio between the weight of adipose tissues and lean tissues, in particular by:
- lowering the weight of adipose tissues in animals as compared to the weight of adipose tissues of animals fed with a normal diet, and
- maintaining or increasing the weight of lean tissues as compared to the weight of lean tissues of animals fed with a normal diet,
by the administration of nutraceutics or food compositions comprising at least one hormone chosen among:
- 3',5',3-triiodothyronine (rT3),
- a rT3 derived hormone, such as 3',3-diiodothyronine, 3',5'-diiodothyronine, 5',3- diiodothyronine, 3'-iodothyronine, 5'-iodothyronine or 3-iodothyronine, or
- a precursor of rT3, such as T4 in association with a molecule susceptible to promote the formation of rT3.

### DRAWINGS

### Figures 1A and 1B

Growth rate of Wistar rats treated wit a high dosage of rT3 or 3,3'-T2 (25 µg/100 g of body weight (BW))
Figures 1A and 1B represent the weight of the rats (in grams) relative to time (in days) for a period of 21 days. The weight of the rats treated with thyroid hormones is shown on the curve with white rectangles and the weight of those treated with placebo is represented with black diamonds.
Figure 1A: the rats were treated with rT3.
Figures 1B: the rats were treated with 3,3'-T2.

### Figures 2A and 2B

Food intake of Wistar rats treated wit a high dosage of rT3 or 3,3'-T2 (25 µg/100 g BW)
Figures 2A and 2B represent the food intake in grams / day of the rats relative to time (in days) for a period of 21 days. The food intake of the rats treated with thyroid hormones is shown on the curve with white rectangles and the food intake of those treated with placebo is represented with black diamonds.
Figure 2A: the rats were treated with rT3.
Figure 2B: the rats were treated with 3,3'-T2.

### Figures 3A and 3B

Energy expenditure of Wistar rats treated wit a high dosage of rT3 or 3,3'-T2 (25 µg/100 g BW)

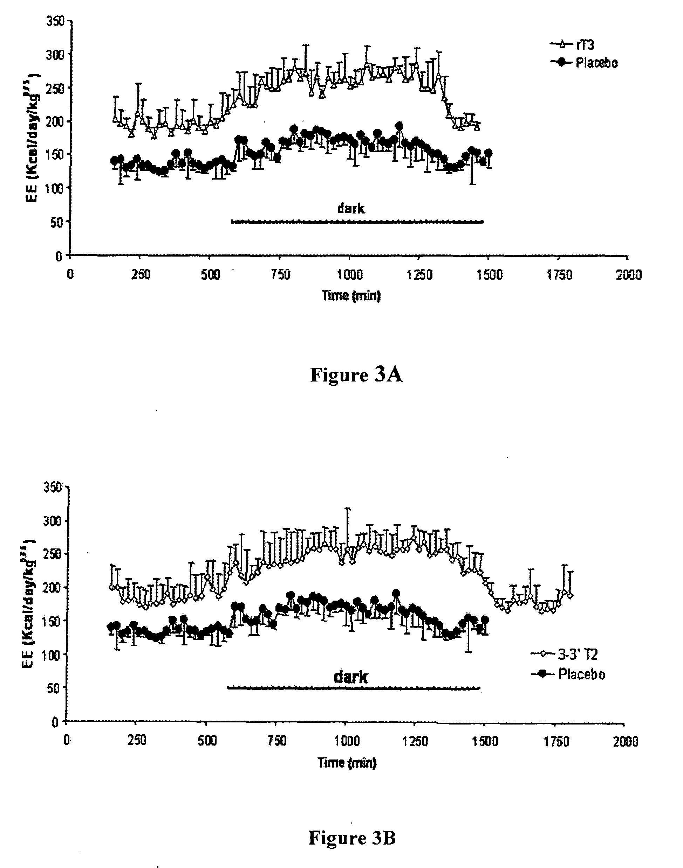

Figures 3A and 3B represent the energy expenditure (EE) in Kcal / day/kg^{0,75} of the rats relative to time (in minutes). The energy expenditure of the rats treated with thyroid hormones is shown on the curve with white triangles (Figure 3A) or white diamonds (Figure 3B) and the energy expenditure of those treated with placebo is represented with black circles.

The horizontal black line indicates a period where the rats are in the dark.
Figure 3A: the rats were treated with rT3.
Figure 3B: the rats were treated with 3,3'-T2.

### Figures 4A and 4B

Respiratory quotient (RQ) of Wistar rats treated with a high dosage of rT3 or 3,3'-T2 (25 µg/100 g BW).

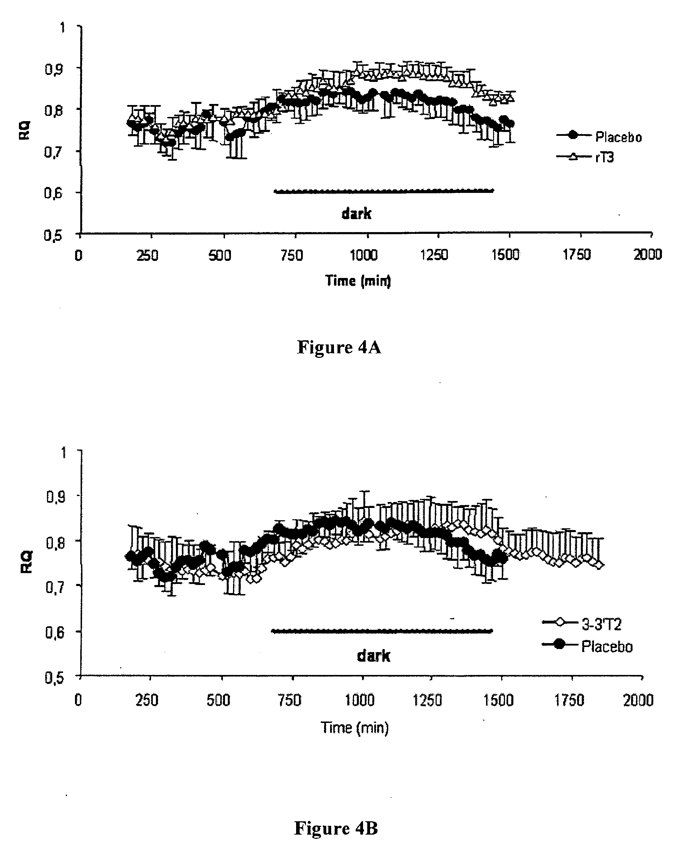

Figures 4A and 4B represent the respiratory quotient of the rats relative to time (in minutes). The respiratory quotient of the rats treated with thyroid hormones is shown on the curve with white triangles (Figure 4A) or white diamonds (Figure 4B) and the respiratory quotient of those treated with placebo is represented with black circles.

The horizontal black line indicates a period where the rats are in the dark.
Figure 4A: the rats were treated with rT3.
Figure 4B: the rats were treated with 3,3'-T2.

### Figures 5A, 5B and 5C

Weight and relative weight of adipose tissues, skeletal muscles and brown adipose tissue of Wistar rats treated with a high dosage of rT3 (250 µg/kg BW).

The results of the rats treated with thyroid hormones are shown in white and the results of those treated with placebo in black.

The asterisk corresponds to a p-value <0.01.

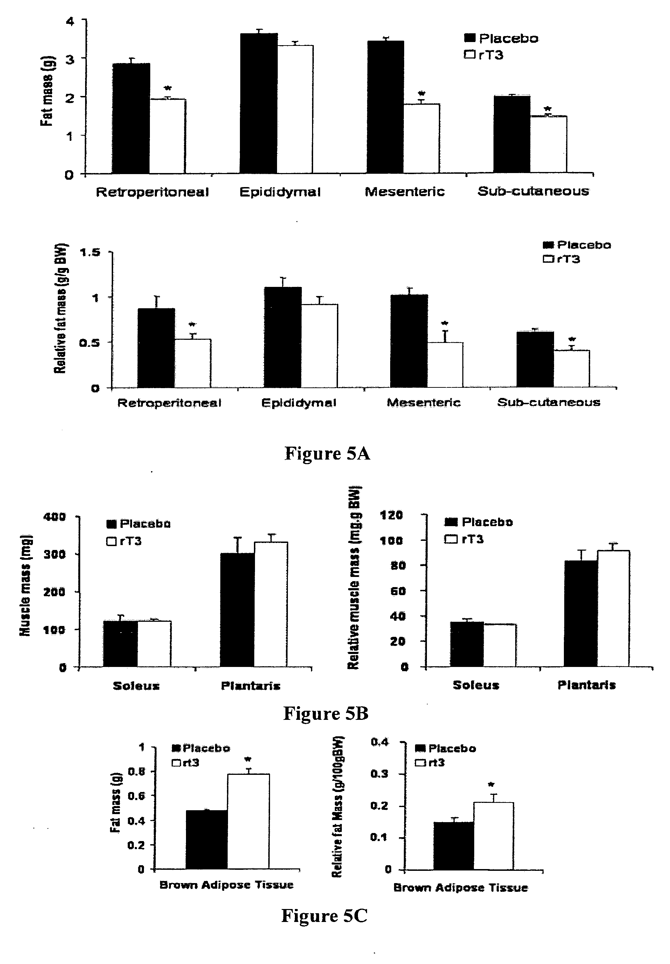

Figure 5A: the upper panel gives the weight (g) of different adipose tissues (retroperitoneal, epididymal, mesenteric and subcutaneous fat) and the lower panel gives the relative weight (g/100 g BW) of these adipose tissues.
Figure 5B: the left panel gives the weight (mg) of skeletal muscles (soleus and plantaris muscles) and the right panel gives the relative weight (mg/100 g BW) of these muscles.
Figure 5C: the left panel gives the weight (g) of interscapular brown adipose tissue and the right panel gives the relative weight (g/100 g BW) of this tissue.

### Figures 6A, 6 B and 6C

Weight and relative weight of adipose tissues, skeletal muscles and brown adipose tissue of Wistar rats treated with a high dosage of 3,3'-T2 (250 µg/kg BW).

The results of the rats treated with thyroid hormones are shown in white and the results of those treated with placebo in black.

The asterisk corresponds to a p-value <0.01.

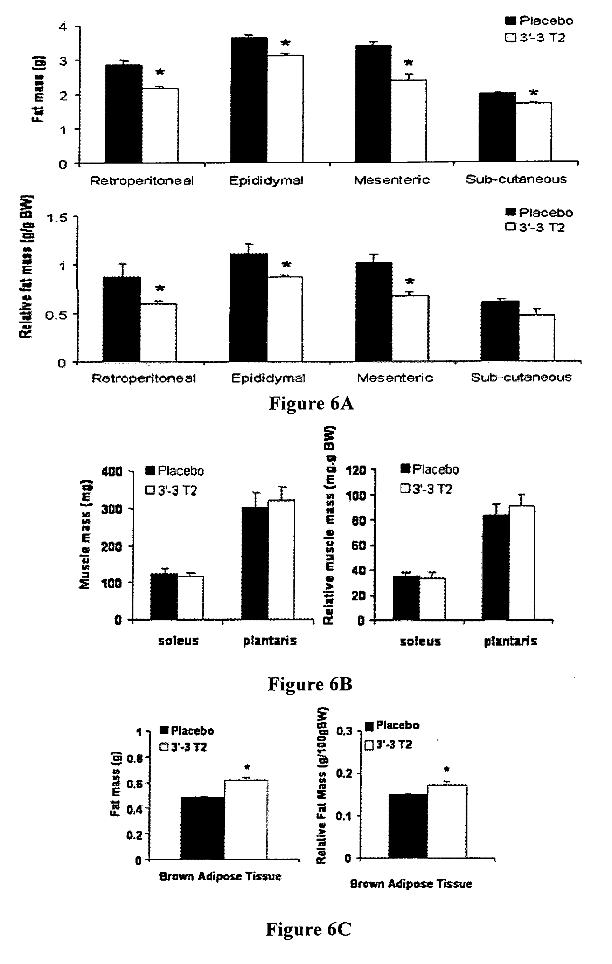

Figure 6A: the upper panel gives the weight (g) of different adipose tissues (retroperitoneal, epididymal, mesenteric and subcutaneous fat) and the lower panel gives the relative weight (g/100 g BW) of these adipose tissues.
Figure 6B: the left panel gives the weight (g) of skeletal muscles (soleus and plantaris muscles) and the right panel gives the relative weight (mg/100 g BW) of these muscles.
Figure 6C: the left panel gives the weight (g) of interscapular brown adipose tissue and the right panel gives the relative weight (g/ 100g BW) of this tissue.

### Figures 7A, 7B, 7C and 7D

Rate of liver mitochondrial oxygen consumption (*J*O₂ in nmol of O₂ / min / mg of protein) of animals treated with a 250 µg/kg BW/day of rT3 or 3,3'-T2.

Measurements were performed using mitochondria (1.0 mg of mitochondrial protein/ml) incubated with various substrates:
- GM: glutamate/malate (5 mM/2.5 mM)
- SR: succinate/rotenone (5 mM/5µM),
- GMS : glutamate/malate/ succinate (5 mM/2.5 mM/5mM),
- Palm: palmitoyl carnitine (55µM),
- Octa: octanoyl carnitine (100 µM),
- TMPD/ascorbate (0.5 mM/0.5 mM) and
- TMPD/ascorbate/DNP (0.5 mM/0.5 mM/75 µM) OK

JO₂ was recorded in the presence of the substrate and following the addition of 1mM ADP (adenosine diphosphate) (state 3).

The oligomycin was added to the mitochondrial suspension to determine the non-phosphorylating respiratory rate (state 4).

Oxygen consumption of rats treated with thyroid hormones is shown in white, and oxygen consumption of those treated with placebo in black.

The asterisk corresponds to a p-value <0.01.

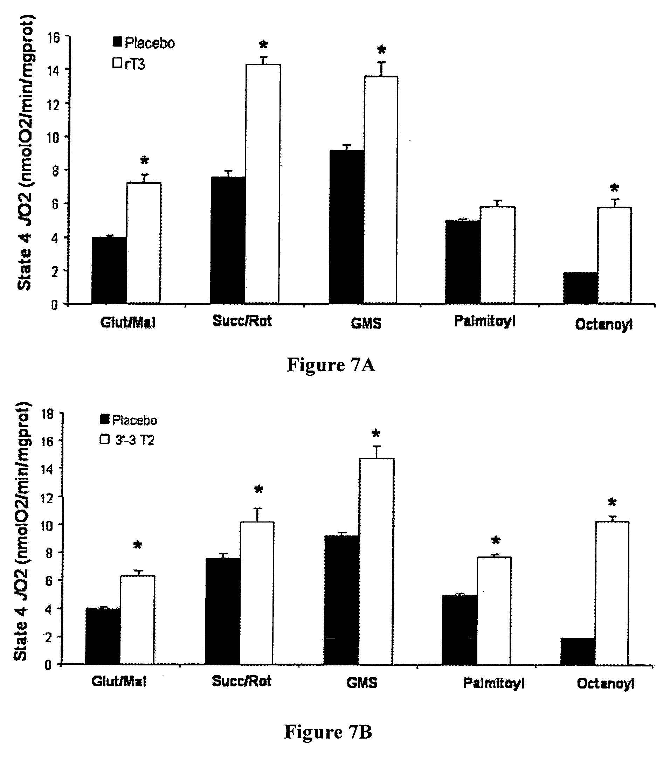

Figure 7A: results obtained with rats treated with rT3 at state 4.
Figure 7B: results obtained with rats treated with 3,3'-T2 at state 4.

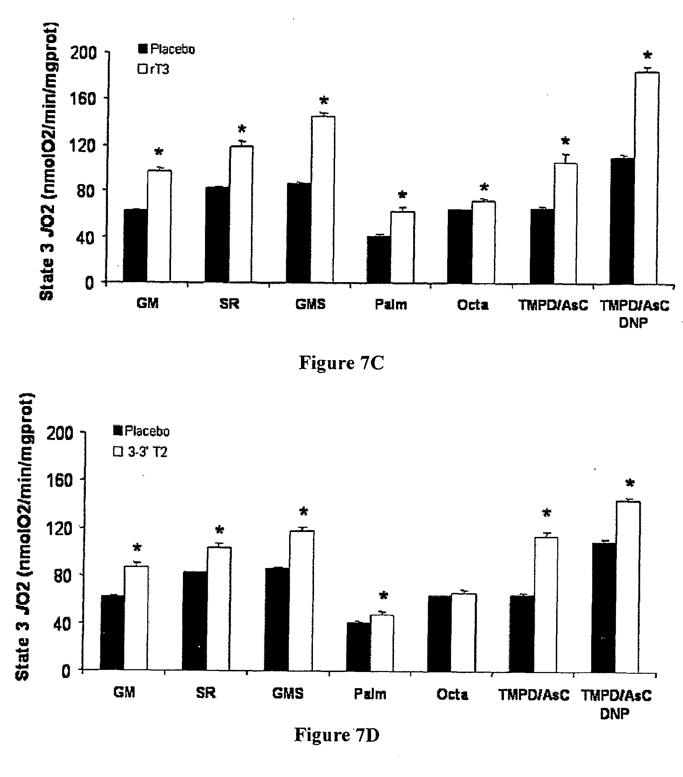

Figure 7C: results obtained with rats treated with rT3 at state 3.
Figure 7D: results obtained with rats treated with 3,3'-T2 at state 3.

### Figures 8A, 8B, 8C and 8D

Rate of muscle mitochondrial oxygen consumption (*J*O₂ in nmol of O₂ / min / mg of protein) of Wistar rats treated with 250 µg/kg BW/day of rT3 or 3,3'-T2.

All measurements were performed using mitochondria (0.2 mg of mitochondrial protein/ml) incubated with various substrates:
- GM: glutamate/malate (5 mM/2.5 mM)
- SR: succinate/rotenone (5 mM/5µM),
- GMS : glutamate/malate/ succinate (5 mM/2.5 mM/5mM),
- Palm: palmitoyl carnitine (55µM), and
- Octa: octanoyl carnitine (100 µM).

JO₂ was recorded in the presence of the substrate and following the addition of 1mM ADP (state 3).

The oligomycin was added to the mitochondrial suspension to determine the non-phosphorylating respiratory rate (state 4).

Oxygen consumption of rats treated with thyroid hormones is shown in white, and oxygen consumption of those treated with placebo in black.

The asterisk corresponds to a p-value <0.01.

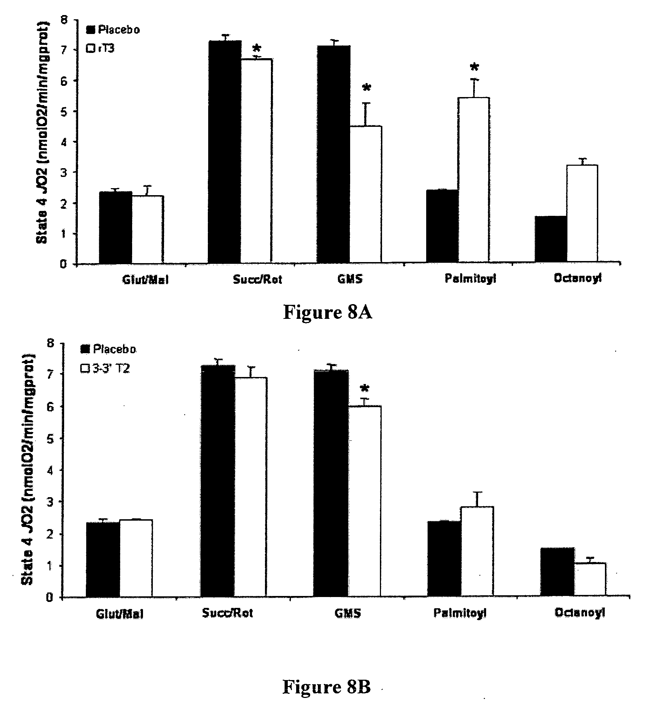

Figure 8A: results obtained with rats treated with rT3 at state 4.
Figure 8B: results obtained with rats treated with 3,3'-T2 at state 4.

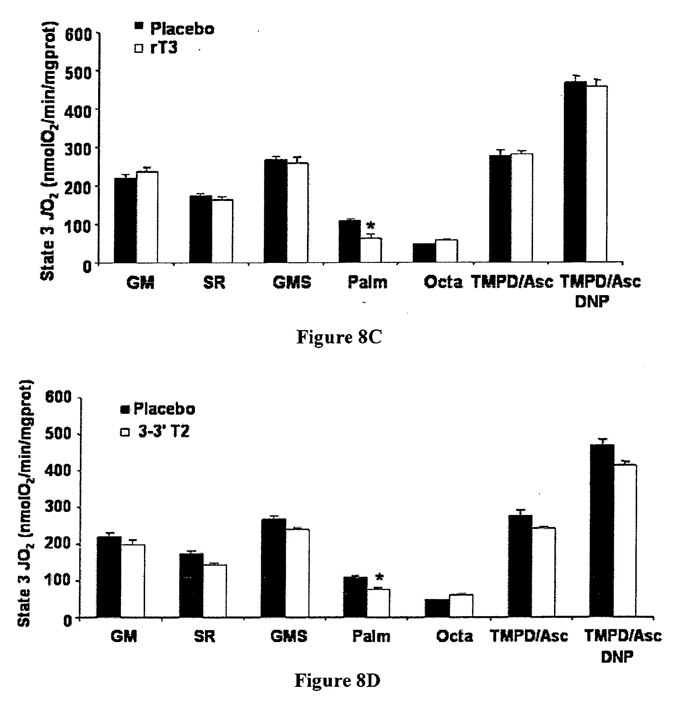

Figure 8C: results obtained with rats treated with rT3 at state 3.
Figure 8D: results obtained with rats treated with 3,3'-T2 at state 3.

### Figures 9A and 9B

Rate of liver mitochondrial oxygen consumption (*J*O₂ in nmol of O₂ / min / mg of protein) of Wistar rats treated with a low dosage of rT3 (25µg/kg BW/day).

Oxygen consumption of rats treated with thyroid hormones is shown in white, and oxygen consumption of those treated with placebo in black.

All measurements were performed using mitochondria (1.0 mg of mitochondrial protein/ml) incubated with various substrates:
- GM: glutamate/malate (5 mM/2.5 mM),
- SR: succinate/rotenone (5 mM/5µM),
- GMS : glutamate/malate/ succinate (5 mM/2.5 mM/5mM),
- Palm: palmitoyl carnitine (55µM),
- Octa: octanoyl carnitine (100 µM),
- TMPD/ascorbate (0.5 mM/0.5 mM) and
- TMPD/AsC/DNP: TMPD/ascorbate/DNP (0.5 mM/0.5 mM/75 µM)

The asterisk corresponds to a p-value <0.01.

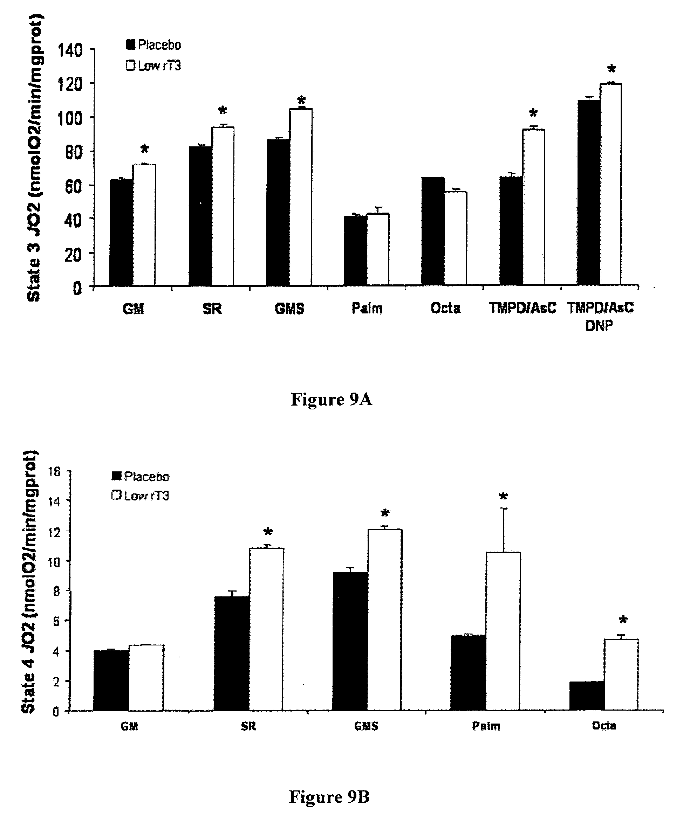

Figure 9A: *J*O₂ was recorded in the presence of the substrate and following the addition of 1mM ADP (state 3).
Figure 9B: *J*O₂ was recorded after the addition of oligomycin to determine the non-phosphorylating respiratory rate (state 4).

### Figures 10A, 10B, 10 C, 10D and 10E

Plasma concentrations of glucose, triglycerides, cholesterol, FFA (Free Fatty Acid) and HDL (Heavy Density Lipoprotein) in Wistar rats treated with 250µg/kg BW/day of rT3 or 3,3'-T2. These measurements were done on venous blood of the rats the day of the sacrifice.

The results of the rats treated with rT3 are shown in white, the results of those treated with 3,3'-T2 in grey and the results of those treated with placebo in black.

The asterisk corresponds to a p-value<0.01.

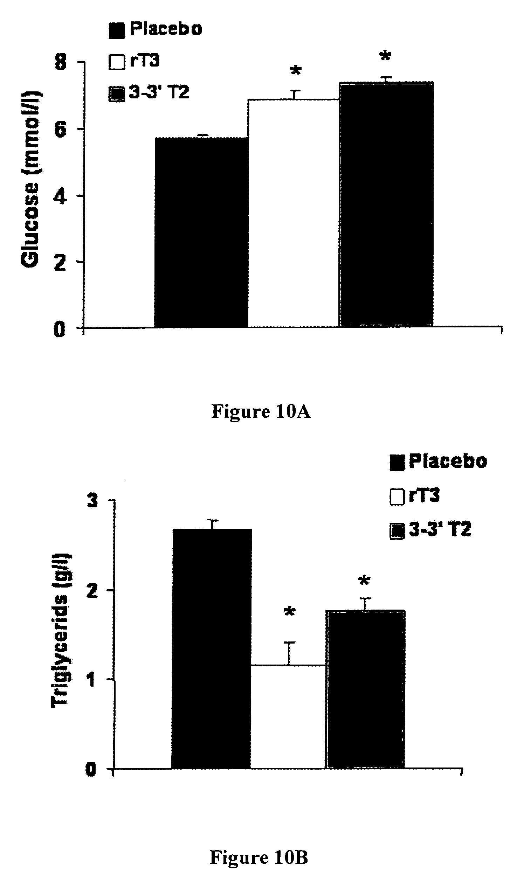

Figure 10A: glucose (mmol/l)
Figure 10B: triglycerides (TG) (g/l)

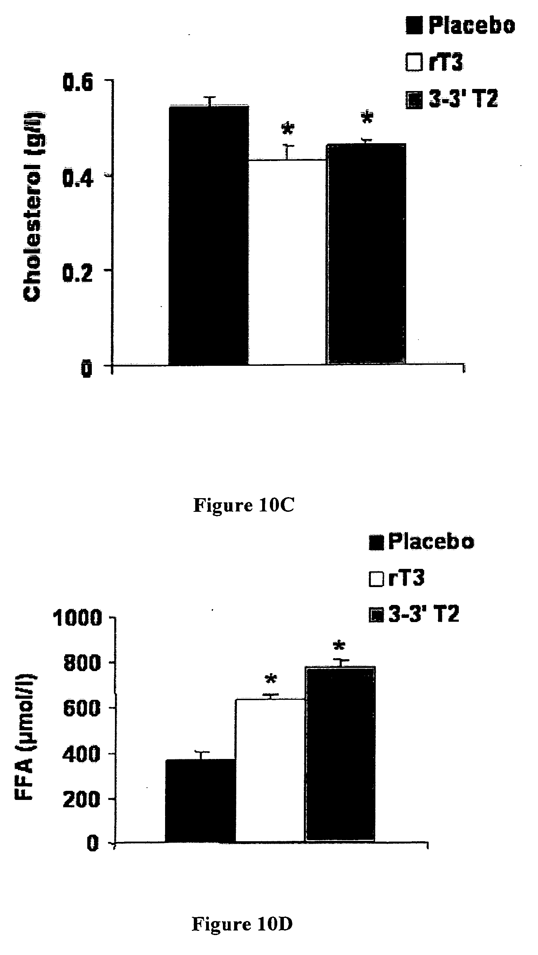

Figure 10C: cholesterol (g/l)
Figure 10D: FFA (µmol/l)

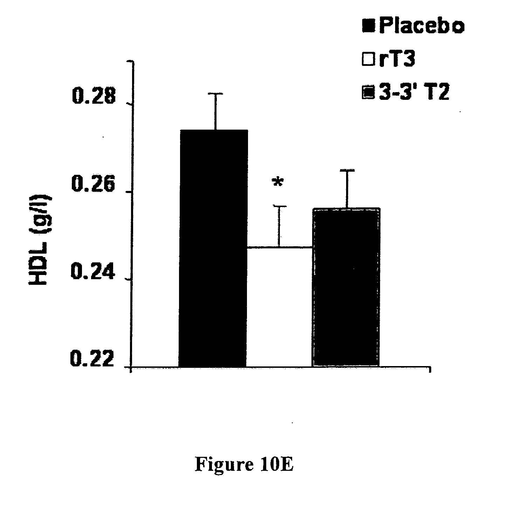

Figure 10E: HDL (g/l)

### EXAMPLES

### Example 1: Use of the rT3 hormone or of a rT3 derived hormone for the treatment of obesity and dyslipidemia

### 1. Material and Methods

### Animal handling

Adult male rat bred in the animal room facilities of Laboratory of Fundamental and Applied Bioenergetics (Wistar strain) or purchased from Charles-River Laboratories, Domaine des oncins, L'ARBRESLE France [(genetically obese normoglycemic (Zucker or Fa/Fa) or diabetic (ZDF)] were caged individually in stainless steel hanging cages and maintained in a 22°C, 50 ± 10% relative humidity and 12h:12h light:dark environment. All animals were fed *ad libitum* with a standard rat chow (Safe A04, Villemoisson, France) and tap water. Body mass and food intake were recorded twice/thrice a week and fresh food was provided at the same time to ensure minimal disturbance to the animals' food behavior.

### Pellet implant

Eight-week old rats (300g±10g) were anesthetized by simultaneous intraperitoneal injection of diazepam 4 mg/kg and ketamine 100 mg/kg. In order to maintain body temperature during the surgery (10 min), animals were placed on a warm blanket. After interscapular shaving, a small incision of 0.5 cm of the skin allows the subcutaneous implantation of a small pellet (containing rT3 or 3',3-T2) with a 10-gauge precision trochar. The pellets, manufactured by Innovative Research of America (Sarasota, Florida, USA) are constituted of a biodegradable matrix that effectively and continuously release the active product in the animal.

3,3',5 triiodo-thyronine (reverseT3) or 3-3'diiodothyronine (3-3' T2) were used at different doses (5, 0.5, or 0.1mg/pellet) were implanted in order to provide a continuous and constant drug delivery over 60 days (which represents 25µg, 2,5 µg or 0.5 µg/day /100g BW).

### Indirect calorimetry

Energy expenditure as well as the nature of substrate oxidized (carbohydrates or lipids) were investigated by indirect calorimetry. This principle is based on the determinations of CO₂ release (VCO₂) and O₂ consumption (VO₂) by each animal. It assumes that O₂ is entirely involved in substrate oxidation in the respiratory chain (leading to water production) while CO₂ release is related to substrate decarboxylation (in the Krebs' cycle). These measurements allow assessing energy expenditure (EE) and respiratory quotient (VO₂/VCO₂, RQ). EE represent the absolute energy dissipation during rest and activity. RQ is a relative measurement indicating the ratio of carbohydrate versus lipid involved in oxidative pathway. A ratio of 1.0 indicates exclusive carbohydrate oxidation while a ratio of 0.7 indicates exclusive lipid oxidation. Each value between these two extreme values indicates the relative proportion of each substrate (of note protein oxidation was not evaluated). As an example, RQ approaches 0.7 during fasting, indicating lipid oxidation, conversely after feeding RQ increases close to 1 indicating carbohydrate oxidation resulting from food intake and blood insulin rise. Likewise, animals fed high-carbohydrate diets have higher RQs than those fed high-fat diets.

The indirect calorimetry system (Panlab, Barcelona, Spain) consists of cages, pumps, flow controllers, valves, and analyzers. It is computer-controlled in order to sequentially measure O₂ and CO₂ concentrations as well as air flow in four separate cages allowing four simultaneous determinations. Rats are isolated in one of the four metabolic chambers, and room air is used as a reference to monitor ambient O₂ and CO₂ concentrations periodically.

At predefined intervals, the computer sends a signal to store differential CO₂ and O₂ concentrations, flow rate, allowing computing VCO₂, VO₂, RQ, and EE (Weir equation) with data acquisition hardware (Metabolism, Panlab, Barcelona, Spain).

### Body composition, blood and tissue sampling

At the end of the experimental period, animals were sacrificed by decapitation, in order to avoid the well-known effects of general anesthetics on mitochondrial metabolism. Blood samples were immediately collected and plasma was frozen for subsequent determination of serum metabolites and hormones. Liver, muscles and fat depots were quickly excised and weighed. Liver median lobe was rapidly freeze-clamped. Muscles (plantaris, soleus and gastrocnemius) were frozen in isopentane precooled in liquid nitrogen. Mesenteric fat consisted of adipose tissue surrounding the gastro-intestinal tract from the gastro-oesophageal sphincter to the end of the rectum with special care taken in distinguishing and removing the pancreas. Retroperitoneal fat pad was taken as the distinct depot behind each kidney along the lumbar muscles. Epididymal fat consisted of adipose tissue on top of the epididymis. For subcutaneous depot measurement, a rectangular piece of skin was taken on the right side of each animal from the median line of the abdomen between the spine and the right hip to the first rib. Interscapular brown adipose tissue was removed and dissected free from adjacent muscles and white adipose tissue. The heart ventricles, the right kidney and the spleen were also excised, weighed and frozen.

### Mitochondrial isolation

The major part of the liver and the red part of each quadriceps were rinsed, and chopped into isolation medium (250 mM sucrose, 20 mM Tris-HCl and 1 mM EGTA-Tris, pH 7.4). Nuclei and cell debris were removed by centrifugation at 800 g for 10 min. Mitochondria were then isolated from the supernatant by spinning twice at 8,000 g for 10 minutes. The mitochondrial pellet was resuspended in 0.5 ml of isolation buffer and kept on ice. Mitochondrial protein was measured by the bicinchoninic acid method (Pierce, Rockford, Illinois). The final mitochondrial suspensions were maintained on ice and were used for measurements of oxygen consumption rate.

### Mitochondrial oxygen consumption

The rate of mitochondrial oxygen consumption (*J*O₂) was measured at 30°C in an incubation chamber with a Clark-type O₂ electrode filled with 2 ml of incubation medium (125 mM KCl, 10 mM Pi-Tris, 20mM Tris-HCl, 0.1 mM EGTA, pH 7.2). All measurements were performed using mitochondria (1.0 or 0.2 mg mitochondrial protein/ml for liver and skeletal muscle) incubated either with various substrates: glutamate/malate (5 mM/2.5 mM) and succinate (5 mM), alone or in combination, palmitoyl carnitine (55µM) and octanoyl carnitine (100 µM). For each substrate, *J*O₂ was recorded in the presence of the substrate alone (State 2) and following the addition of 1mM ADP (state 3). Oligomycin (1.25µg/mg protein) was added to the mitochondrial suspension to determine the non-phosphorylating respiratory rate (state 4). The incubation medium was constantly stirred with a built-in electromagnetic stirrer and bar flea. The efficiency of the mitochondrial oxidative phosphorylation was assessed by the state 3/state 4 ratio which measures the degree of control imposed on oxidation by phosphorylation (respiratory control ratio, RCR).

### Oxidative phosphorylation efficiency

ATP/O ratios with 5 mM glutamate/2.5 mM malate/5 mM succinate or octanoyl-carnitine (100 µM) as respiratory substrates were determined from the ATP synthesis rate (*J*_{ATP}) versus respiratory rate *J*O₂ with an ADP regenerating system based on hexokinase (EC 2.7.1.1) plus glucose. *J*_{ATP} and *J*O₂ were measured as described above in a medium containing 125 mM KCl, 1 mM EGTA, 5 mM Tris-Pi, 20 mM Tris-HCl, 0.1% fat free BSA (pH 7.2). *J*_{ATP} was determined from glucose 6-phosphate formation in presence of 20 mM glucose, 1 mM MgCl₂, and 125 µM ATP. *J*O₂ and *J*_{ATP} were modulated by addition of increasing concentrations of hexokinase (Nogueira et al, J Bioenerg Biomemb., 34: 55-66, 2002*).*

### Enzymatic activities

Measurement of the specific activity of the respiratory-chain complex I, II and IV was performed spectrophotometrically. A total of 8-10 µg of mitochondrial proteins were required to determine the activity of complex I and II, and 4 µg were used for complex IV. Enzyme activity was expressed as nmoles of reduced or oxidized substrate per min and per mg of mitochondrial protein.

*Measurement of complex I (rotenone-sensitive NADH-ubiquinone oxidoreductase, EC 1.6.99.3):* The assay was performed using decylubiquinone (100 µM) as electron acceptor and NADH (200 µM) as a donor, in a 10 mM KH₂PO₄/K₂HPO₄ buffer (pH 7.5) containing BSA (3.75 mg/ml), and in the presence of KCN (2 mM) and antimycin A (7.5 µM). The oxidation of NADH was then measured at 340nm before and after the addition of rotenone (4 µM), allowing the calculation of the rotenone-sensitive specific activity of complex I.

*Measurement of complex II (succinate-ubiquinone reductase, EC 1.3.99.1):* Succinate-ubiquinone oxidoreductase activity was quantified by measuring the decrease in UV absorbance due to the reduction of DCPIP (100 µM) at 600 nm. The measurement was performed in a medium containing 50 mM KH₂PO₄/K₂HPO₄ (pH 7.5) in the presence of decylubiquinone (100 µM), rotenone (2 µM) and KCN (2 mM).

*Measurement of complex IV (cytochrome c oxidase, EC 1.9.3.1):* The assay was performed by measuring cytochrome c (100 µM) oxidation at 550nm in a 50 mM KH₂PO₄/K₂HPO₄ buffer (pH 7.0).

*Citrate synthase* activity was determined by measuring the UV absorbance at 412nm due to the formation of the ion mercaptide in the presence of oxaloacetate dinitrothiobenzoïque acid and acetyl-CoA in a 150 mM Tris buffer pH 8 (Garait et al, Free Rad Biol Med, 2005).

*Mitochondrial glycerol 3-phosphate dehydrogenase (mGPdH)* activity was measured on the supernatant of isolated mitochondria after three cycles of freezing-thawing. Forty µg of mitochondria were incubated in a KH₂PO₄/K₂HPO₄ buffer (50 mM, pH 7.5) containing 9.3 µM of antimycin A, 5µM of rotenone and decylubiquinone (50µM). The reduction of 50 µM dichloro-indophénol (DCIP) by mGPDH was measured spectrophotometrically at 600 nm at 37°C and enzymatic activity was expressed as µmol.min⁻¹.mg prot⁻¹.

### Cytochromes

Cytochromes content of the mitochondrial respiratory chain was measured in parallel experiments by comparing the spectra of fully oxidized (potassium ferricyanide) *versus* fully reduced (few crystals of sodium dithionite) cytochromes. Knowing the contributions in absorbance of each cytochrome to the major maxima and minima of each of the other cytochromes, a set of 4 simultaneous equations with 4 unknowns can be derived and concentration of each cytochrome can be calculated *(*Williams, Arch Biochem Biophys.; 107: 537-43, 1964).

### Hepatocytes Isolation

Wistar rats fasted for 20-24 h were anesthetized with sodium pentobarbital (10 mg/100 g body wt i.p.), and the hepatocytes were isolated according to the method of Berry and Friend (J. Cell. Biol. 43: 506-520, 1969) as modified by Groen et al. (Eur. J Biochem. 122: 87-93, 1982). Briefly, the portal vein was cannulated, and a 2-min anterograde perfusion with Ca²⁺-free Krebs-Ringer bicarbonate buffer (25 ml/min; 37°C, pH = 7.4, continuously gassed with 95% O₂-5% CO₂) was performed to remove blood from the liver. A 10-min retrograde perfusion (25 ml/min) through the posterior vena cava was started with the same perfusion medium. Subsequently, a recirculating perfusion was performed (20 min at 40 ml/min) with 100 ml Krebs-Ringer medium supplemented with 0.25 mg/ml collagenase (type IV, Sigma, St. Louis, MO). The liver was then cut and shaken in the perfusion medium for 2 min under constant gassing (95% O₂-5% CO₂). Finally, the cell suspension was filtered through nylon gauze (pore size, 120 µm), washed twice with Krebs-Ringer bicarbonate buffer containing 1.6 mM Ca²⁺, and then washed for a third time with the same buffer supplemented with 1% BSA.

### Perifusion of hepatocytes

Liver cells were perifused according to the method of van der Meer and Tager (FEBS Lett. 67: 36-40, 1976) modified by Groen et al. (Eur. J Biochem. 122: 87-93, 1982). Hepatocytes (225-250 mg dry mass) were placed in 15-ml perifusion chambers at 37°C and were perifused (5 ml/min) with a continuously gassed (95% O₂-5% CO₂) Krebs-Ringer bicarbonate solution (pH = 7.4) containing 0.2% BSA. The experiments were carried out in duplicate in two perifusion chambers placed in parallel. At the chamber outlet, perifusate O₂ content was monitored with Clark electrodes (Yellow Springs Instruments, Yellow Springs, OH) to assess O₂ uptake of the hepatocyte suspension. After 40 min , when O₂ uptake had reached a steady state, hepatocytes were perifused with increasing amount of glycerol (0.15, 0.30, 0.60,1.2, 2.4, 4.8, and 9.6 mM), in the presence or not of 0.4 mM octanoate. At the end of each steady state of 20 min, perifusate and cells samples were collected at 2-min intervals for subsequent determination of glucose, lactate, pyruvate, acetoacetate, and P-hydroxybutyrate concentrations. Samples were stored at 4°C and analyzed within 12 h after the end of the experiment. In addition, 300 µl of the cell suspension were sampled from the chamber for intra- and extracellular fractionation. For this purpose, mitochondrial and cytosolic spaces were separated with the digitonin fractionation method described by Zuurendonk and Tager (Biochim. Biophys. Acta 333: 393-399, 1974). Briefly, the cell suspension was placed in a 2.2-ml Eppendorf tube in an isotonic medium containing 2 mM of digitonin (Merck, Lyon, France) at 4°C. After 15 s, the tube was centrifuged for 15 s at 10,000 g to precipitate mitochondria through the underlying 800-µl layer of silicon oil (Rhodorsil 640 V 100, Rhône-Poulenc) into 250 µl HClO₄ (10% mass/vol) + 25 mM EDTA. The supernatant (700 µl) was immediately removed, deproteinized with HClO₄ (5% mass/vol), and neutralized. The intracellular content was then neutralized and kept at -20°C for determination of intracellular metabolites (DHAP and G3P, spectrophotometry) and adenine nucleotides content (HPLC).

### Western blot analysis

For mGPdH quantification, polyacrylamide gel electrophoresis and immunoblotting were performed as previously described (23). Briefly, lysed hepatocytes were mixed with 200 µl of buffer containing 40 mM Tris(hydroxymethyl)aminomethane pH 6.8, 1% SDS, 6% glycerol, and 1% b-mercaptoethanol. This mixture was then heated at 100°C for 10 min, and subjected to one -dimensional sodium dodecyl sulfate (SDS)-PAGE with a 5% stacking and 12.5% resolving gels for 12 hours. After electrophoretic separation, proteins were transferred at a constant voltage to PVDF membranes. After protein transfer, the membranes were blocked for 2h, then incubated 2 h with a monoclonal antibody specific for mGPDH (generous gift from Dr. J. Weitzel) and then exposed to the secondary antibody (goat antimouse immunoglobulin G conjugated to horseradish peroxidase, Bio-Rad at a 1:10000 dilution). mGPDH were visualized by the enhanced chemiluminescence detection method (RPN 2106, Amersham). Scanning with a densitometer performed quantification of bands from blots and the data were expressed numerically as integrated optical density arbitrary units.

### RNA purification and Reverse Transcription-coupled PCR

Total RNA were extracted from tissue using Tripure RNA Isolation reagent (Roche Diagnostics). Concentration and purity were verified by measuring optimal density at 260 and 280 nm. Their integrity was checked by 1% agarose gel electrophoresis (Eurobio). mRNA concentrations were measured by semi-quantitative reverse transcription polymerase chain reaction (RT-PCR) using β actin as reference. Primer sequences are shown in table 1. For each target mRNA, a RT was performed from 0.1 µg of total RNA with 100 U of M-MLV Reverse Transcriptase (Promega), 5µL of M-MLV RT 5X buffer, 20 U of RNasin Ribonuclease Inhibitor, 12 picomoles of deoxynucleoside triphosphate and 15 picomoles of the specific antisense primer, in a final volume of 25 µL. The reaction consisted in 5 min at 70°C (RNA and antisense primer), then 60 min at 42°C (all mix) followed by 15 min at 70°C. After chilling, 5 µL were used for PCR reaction. The 5 µL of RT medium were added to 45 µL of PCR mix (5 µL 10X REDTaq PCR buffer) containing 6 picomoles of MgCl2, 8 picomoles of deoxynucleoside triphosphate, 2.5 U of REDTad DNA polymerase (Sigma), 15 picomoles of corresponding antisense primers and 22.5 picomoles of sense primers. The PCR conditions were: 2 min at 94 °C followed by 28 cycles, 35 cycles or 18 cycles for UCP3, UCP2 and β actin respectively (1 cycle = 1 min at 94°C, 1 min at 60°C, 1 min at 72°C). PCR was ended by 10 min at 72°C. Products were analysed on 2% agarose gel prestained with ethidium bromide. For quantitation of relative bands intensities, pictures were taken with a Camera DC120 (Kodak) and the ratio of each target to β actine was determined for each sample with Kodak Digital Science 1D 2.0 (Kodak Scientific Imaging System).

### 2. Results

As shown in Figures 1 (A and B), control (placebo treated) Wistar rat body exhibit a normal growth rate of 150 g over 21 days (*i.e.* a weight gain of about 40%). Treated animals with either rT3 (Figure 1A) or 3,3'-T2 (Figure 1B) did not show any weight gain, the body mass after 21 days being not significantly different from the initial value.

This indicates a very powerful prevention of normal weight gain in these young adult animals.

As shown in Figures 2 (A and B), the food intake of placebo group was stable over the experimental period around 30 g of food per day. Both groups of treated animals showed similar changes a decrease in food intake immediately after the pellet, containing rT3 (Figure 1A) or 3,3'-T2 (Figure 1B), have been introduced subcutaneously (from day 4 until day 7) then food intake increase showing higher value as compared to those of placebo animals.

Hence the decrease in body weight in both groups of treated animals was associated with an increased food intake.

The energy expenditure (EE) of rats was assessed by indirect calorimetry (see material and method section) and values were analyzed over a period of 24 hours (= 1440 minutes). All groups of animals, treated with placebo (Figures 3A and 3B), rT3 (Figure 3A) or 3,3'-T2 (Figure 3B), exhibited days/nights variations due the classical nocturnal activity and eating behavior of these rodents contrasting with the quiet diurnal period. Both groups of treated rats exhibited a dramatic increase in energy expenditure reaching 25 to 30 of the control values.

This very important result indicates that the metabolic expenses are largely increased by the two treatments both during the nocturnal and the diurnal periods.

The respiratory quotient (RQ) is defined as the ratio between released carbon dioxide to consumed oxygen: VCO₂/VO₂. It is largely accepted that this ratio indicate the origin of oxidized substrates (carbohydrate versus lipids). This value is equal to 1 if carbohydrates represent the exclusive source of energy and 0.7 were lipids represent the unique energetic substrate.

As already shown the EE, RQ also varies between day and night (Figures 4A and 4B). It is higher during the night, when animals are eating and therefore oxidizing more carbohydrates. Conversely during the diurnal period RQ is lower indicating a fasting sate were lipids are the predominant substrates. Regarding rT3 (Figure 4A), it appears that RQ is almost identical to placebo during the day and higher during the night. This would indicate either a higher proportion of carbohydrate or, more likely, a net lipid synthesis from carbohydrate (leading to a RQ value higher than 1) the value presented by these animals being the sum of substrate oxidation and substrate (lipid synthesis) during the fed state. These changes are quite substantial as compared to placebo. In the 3,3'-T2 group (Figure 4B) the changes during the night were almost the same as those described for rT3 and indicates also most probably a net lipid synthesis during the fed state. However during the diurnal period, or immediately after the dark it seems that the RQ is lower than that of placebo indicating a higher fat oxidation in these fasting animals.

The change in body composition of rats treated with rT3 or placebo are presented both as absolute values (g) or as percentage of total body mass since the two groups of animals did not exhibit the same mass after three weeks (see Figure 1A).

Retroperitoneal, mesenteric and subcutaneous fat masses were significantly lower (p<0.01) in rT3 group as compared to placebo, epididymal mass being not different (Figure 5A). This difference was very substantial whatever the data are expressed as absolute or relative values. Interestingly muscle mass was not affected at all (Figure 5B), while brown adipose tissue, a tissue known to be involved in metabolic efficiency and heat production, was significantly increased in rT3 treated animals (Figure 5C).

These results clearly indicate that the decrease in body mass after rT3 treatment is purely due to a loss of fat mass, the lean body mass being not affected.

Similar results are obtained with 3,3'-T2 (Figures 6A and 6B) leading to reach the same conclusion regarding the effect of 3,3'-T2 on fat mass (significantly decreased) and lean body mass (not affected). Interestingly brown adipose tissue was also significantly increased by the treatment.

The effect of both treatments (rT3 or 3,3'-T2) on the efficacy of the coupling between oxidation and phosphorylation at the level of liver mitochondrial respiratory chain were evaluated (Figures 7A and 7B). Respiratory rates of non-phosphorylating mitochondria (i.e. in the presence of oligomycin) of the different groups (rT3, Figure 7A and 3,3'-T2, Figure 7B) of treated animals versus placebo were measured. In both cases (rT3 and 3,3'-T2) respiration was much higher as compared to placebo indicating a less efficiency coupling due to the treatments. The different conditions glutamate/malate, succinate-rotenone, glutamate/malate/succinate, palmitoylCoA, octanoylCoA indicate the different substrates provided to the respiratory chain.

Figures 7C and 7D represent the maximal respiratory rate of liver mitochondria achieved in phosphorylating condition (i.e. in the presence of ADP) with the various substrate supply (see above Figure 7): TMPD ascorbate investigate complex 4 (cytochrome c oxidase) without or with uncoupling state by DNP. Schematically in all conditions the treatments, either with rT3 (Figure 7C) or 3,3'-T2 (Figure 7B), were responsible for a very significant increased respiratory rate indicating that the treatments increased the maximal respiratory capacity for all substrates, including fatty acids.

Very interestingly completely different results were obtained with muscle mitochondria. Indeed both rT3 and 3,3'-T2 failed to substantially affect both non-phosphorylating (state 4, Figures 8A and 8B) and phosphorylating (state 3, Figures 8C and 8D) states. Actually there were some minor effects leading to a decreased respiration, palmytoyl- and octanoyl-CoA excepted.

Hence this indicated that although both rT3 and 3,3'-T2 exhibit a powerful effect on liver mitochondria leading to the a decreased oxidative phosphorylation efficiency and to an increased maximal respiratory capacity, almost no effect was found on muscle mitochondrial despite the fact that the drug was administered to every tissue (subcutaneous progressive release from the pellet).

Figures 9A and 9B show similar data as presented in the figures 7A and C. However animals were treated with a ten fold lower rT3 dose (25µg/kg instead of 250µg/kg in the figure 7). Essentially similar findings were made although to a lower extent. However the decreased efficiency and the higher maximal respiratory capacity are found to be significant.

Figures 10 show the effect of the two treatments on glucose (Figure 10A), triglycerides (Figure 10B), cholesterol (Figure 10C), free fatty acids (Figure 10D) and HDL (Figure 10E) (rats almost do no have LDL) plasmatic concentrations. Both treatments slightly increased fasting glucose in these normal (non diabetic animals) indicating that none of these treatments was responsible for a potential hypoglycemic effect. Interestingly triglycerides and cholesterol were significantly lower with rT3 and 3,3'-T2 as compared to placebo. Plasma fatty acids were higher as it is observed in animals exhibiting a high rate of lipolysis and fatty acid oxidation as it was already suggested by the data obtained with indirect calorimetry.

In conclusion, the dramatic effect observed in the body mass is completely explained by the decreased fat mass, while the lean body mass (muscle mass) seems not affected. This effect, which is observed despite increased food consumption, is due to an increased energy expenditure, which was substantiated by indirect calorimetry measurement. Since the normal diet of these animals is rather poor in lipid content (4-5%) the increase fat oxidation is achieved at the expense of the fat storage as shown by the strong decrease in fat mass and also probably by a de novo lipogenesis, an expensive pathway, which might explain the higher RQ observed in the fed period. The data concerning the overall increase in energy metabolism (indirect calorimetry) are in very good agreement with the data obtained in liver isolated mitochondria indicating the probably occurrence of energy wasting process at the level of the respiratory chain and ATP synthesis associated with a significant higher maximal respiratory capacity. Most interestingly none of these effects was observed in muscle mitochondria indicating that the wasting process affects more the liver than the muscle mass and concerns lipid oxidation.

Hence in total both rT3 and 3,3'-T2 enhance lipid oxidation and energy expenditure leading to a marked decrease in the mass of adipose tissue only.

### Example 2: Use of the rT3 hormone or of a rT3 derived hormone for the treatment of diabetes

### 1. Material and Methods

The Material and Methods are those described in Example 1.

### Animals

Rats were genetically obese normoglycemic (Zucker or Fa/Fa), 10-11 week-old diabetic rats (ZDF) or genetic non-overweight diabetic (type 2 diabetes) rats (Goto-Kakizaki (GK) model).

### Blood sampling

The day of the study, after a fasted period overnight (18h), blood samplings will be taken in awaked rats from the tail vein.

### Blood parameters

The following biochemical parameters were analyzed: glycemia, insulinemia, HbA1c, TG and Cholesterol.

Thyroid Stimulating Hormone (TSH) and thyroxine (T4) were measured by radioimmunoassay with rat standards (RPA 554 Amersham bioscience, RIA FT4-immunotech, for TSH and T4 respectively).

Insulin levels were determined with commercial kits (Linco Research).

Glucose and 3-hydroxybutyrate (3-HB) were measured enzymatically and non esterified fatty acid (NEFA) by colorimetric assay (Wako Chemicals).

Triglycerides and cholesterol were measured by classical routine automate apparatus.

### 2. Results

GK rats treated with low dosage rT3 (2.5µg/100 g BW) during 10 days exhibit a significant decrease in blood glucose concentration of -38±3% (n=5, p<0.01) as compared to control animals (blood glucose concentration of 2.64±0.21 g/l (n=22)). This decrease indicates an improvement of the hyperglycaemic status of these animals already after 10 days.

## Claims

1. Pharmaceutical composition comprising, as active substance, at least one hormone chosen among:
- 3',5',3-triiodothyronine (rT3),
- a rT3 derived hormone, such as 3',3-diiodothyronine, 3',5'-diiodothyronine, 5',3- diiodothyronine, 3'-iodothyronine, 5'-iodothyronine or 3-iodothyronine, or
- a precursor of rT3, such as T4 in association with a molecule susceptible to promote the formation of rT3,
in association with a pharmaceutically acceptable vehicle.

2. Pharmaceutical composition according to claim 1, wherein said active substance is rT3.

3. Pharmaceutical composition according to any of claims 1 to 2, in a suitable form for the release of about 0.01 µg/kg/day to about 250 µg/kg/day, particularly about 0.01 µg/kg/day to about 25 µg/kg/day, particularly about 0.1 µg/kg/day to about 15 µg/kg/day of active substance, more particularly about 0.1 µg/kg/day to about 5 µg/kg/day of active substance, most particularly about 0.1 µg/kg/day to 1 µg/kg/day of active substance.

4. Pharmaceutical composition according to claim 1 or 3, comprising by dosage unit about 5 µg to about 1.5 g of active substance, particularly about 75 mg to about 750 mg of active substance.

5. Use of at least one hormone chosen among:
- 3',5',3-triiodothyronine (rT3),
- a rT3 derived hormone, such as 3',3-diiodothyronine, 3',5'-diiodothyronine, 5',3- diiodothyronine, 3'-iodothyronine, 5'-iodothyronine, or 3-iodothyronine, or
- a precursor of rT3, such as T4, in association with a molecule susceptible to promote the formation of rT3,
for the preparation of a drug intended for the treatment of:
- hyperglycemia, insulin resistance, beta pancreatic cell insufficiency, or related pathologies,
- pathologies wherein the cholesterol and/or triglycerides plasma concentrations are higher than the normal concentrations, dyslipidemia, or
- pathologies related to overweight or related to an excess of fat deposit.

6. Use according to claim 5, for the preparation of a drug intended for the treatment of pathologies chosen among diabetes, particularly type 1 or 2 diabetes, beta pancreatic cell insufficiency, obesity, overweight or related pathologies, hypercholesterolemia, hypertriglyceridemia, dyslipidemia, alcoholic and non alcoholic hepatic steatosis, atherosclerosis, hepatopathies associated to a dysmetabolism, cholecystopathies, deposit of subcutaneous fat, particularly cellulite or vasomotor rhinitis.

7. Use according to claim 5 or 6, wherein said hormone is rT3.

8. Pharmaceutical composition according any of claims 1 to 4, wherein said pharmaceutically acceptable vehicle refers to pharmaceutically acceptable solid or liquid, diluting or encapsulating, filling or carrying agents, which are usually employed in pharmaceutical industry for making pharmaceutical compositions.

9. Pharmaceutical composition according any of claims 1 to 4 or 8, suitable for an administration via an oral, intravenous, intramuscular, subcutaneous, transcutaneous, nasal, intraperitoneal, sublingual, or rectal route.

10. Pharmaceutical composition according any of claims 1 to 4 or 8 or 9, wherein said pharmaceutically acceptable vehicle allows a continuous, preferably constant, release, of said active substance.

11. Product comprising:
- at least one hormone chosen among 3',5',3-triiodothyronine (rT3), a rT3 derived hormone, such as 3',3-diiodothyronine, 3',5'-diiodothyronine, 5',3-diiodothyronine, 3'-iodothyronine, 5'-iodothyronine, or 3-iodothyronine, or a rT3 precursor, such as T4 in association with a molecule susceptible to promote the formation of rT3,
- at least one active substance activating the pancreatic secretion of insulin, particularly chosen among, or susceptible of slowing the digestive absorption of glucose,
as a combination product for a simultaneous, separate or sequential use intended for the treatment of diabetes.
